# EUROPEAN PATENT APPLICATION

(11) **EP 1 452 178 A1**
(43) Date of publication of application: **01.09.2004**
(21) Application number: 02747708.2
(22) Date of filing: 22.07.2002
(51) Int. Cl.: A61K 31/5575, A61K 9/70, A61P 1/02, A61P 3/14, A61P 19/00, A61P 19/02, A61P 19/08, A61P 19/10, A61P 35/04

(54) **PERSISTENT FILMY PREPARATION FOR TOPICAL ADMINISTRATION CONTAINING PROSTAGLANDIN DERIVATIVE**

(30) Priority: 12.11.2001 JP 2001345678
(71) Applicant: ONO PHARMACEUTICAL CO., LTD., Osaka-shi, Osaka 541-8526 (JP)
(72) Inventor: TABATA, Yasuhiko, Uji-shi, Kyoto 611-0024 (JP); NISHIURA, Akio, c/o Minase Res. Inst. Ono Pharma., Mishima-gun, Osaka 618-8585 (JP)
(74) Representative: Henkel, Feiler & Hänzel
(86) International application number: PCT/JP2002/007386
(87) International publication number: WO 2003/041717

(57) **Abstract**

A sustained release film preparation for local administration to the region of diseases associated with decrease in bone mass, which comprises, as an active ingredient thereof, (11α,13E,15α)-9-oxo-11,15-dihydroxy-16-(3-methoxymethylphenyl)-17,18,19,20-tetranor-5-thiaprost-13-enoic acid methyl ester having the following formula (I) or a non-toxic salt thereof: 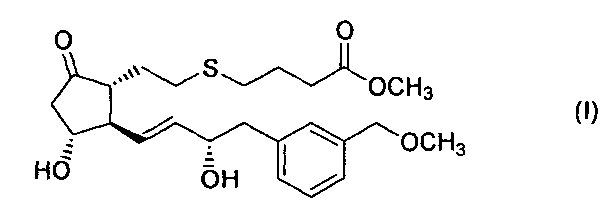 and a film comprising a biodegradable polymer. The sustained release film preparation which comprises the compound (I) as an active ingredient of the present invention has activity of accelerating bone formation and is useful for treatment of diseases associated with decrease in bone mass.

## Description

### TECHNICAL FIELD

The present invention relates to:
(1) a sustained release film preparation for local administration to the region of diseases associated with decrease in bone mass, which comprises, as an active ingredient, (11α,13E,15α)-9-oxo-11,15-dihydroxy-16-(3-methoxymethylphenyl)-17,18,19,20-tetranor-5-thiaprost-13-enoic acid methyl ester (hereinafter referred to as "compound (I)"); and
(2) a therapeutic agent for primary osteoporosis, secondary osteoporosis, metastatic bone, hypercalcemia, Paget's disease, bone loss, bone disease of osteonecrosis, bone formation after operation for bone, or alternative treatment for bone grafting, which comprises a sustained release film preparation for local administration which comprises compound (I) as an active ingredient.

### BACKGROUND ART

From recent studies, it is known that a prostaglandin-E₂ (hereinafter abbreviated to "PGE₂") receptor includes subtypes with different roles. Subtypes known at present are classified into 4 subtypes which are called EP₁, EP₂, EP₃ and EP₄ (Negishi M. *et al*., *L*. *Lipid Mediators Cell Signaling,* 12, 379-391 (1995)).

It is thought that EP₄ receptor relates to inhibition of TNF-α production and acceleration of IL-10 production. Therefore, compounds which bind to EP₄ receptor are expected to be useful for the prevention and/or treatment of immunological diseases (autoimmune diseases such as amyotrophic lateral sclerosis (ALS), multiple sclerosis, Sjoegren's syndrome, chronic rheumarthrosis and systemic lupus erythematosus etc., and rejection after organ transplantation etc.), asthma, neuronal cell death, arthritis, lung failure, pulmonary fibrosis, pulmonary emphysema, bronchitis, chronic obstructive pulmonary disease, liver damage, acute hepatitis, nephritis, renal insufficiency, hypertension, myocardiac ischemia, systemic inflammatory response syndrome, sepsis, hemophagous syndrome, macrophage activation syndrome, Still's disease, Kawasaki disease, burn, systemic granulomatosis, ulcerative colitis, Crohn's disease, hypercytokinemia at dialysis, multiple organ failure, shock, and the like.

Since EP₄ receptor relates to protection of mucosa, compounds which bind to EP₄ receptor are expected to be useful for prevention and/or treatment of ulcer of gastrointestinal tract such as gastric ulcer and duodenal ulcer and stomatitis. Furthermore, since EP₄ receptor relates to physiological sleep induction and platelet aggregation inhibition, compounds which bind to EP₄ receptor are considered to be useful for somnipathy and thrombosis.

In addition, since compounds which bind to EP₄ receptor have activity of promoting bone formation, they are considered to be useful for treatment for diseases associated with decrease in bone mass, such as:
1) primary osteoporosis (e.g., primary osteoporosis followed by aging, postmenopausal primary osteoporosis, primary osteoporosis followed by ovariectomy),
2) secondary osteoporosis (e.g., glucocorticoid-induced osteoporosis, hyperthyroidism-induced osteoporosis, immobilization-induced osteoporosis, heparin-induced osteoporosis, immunosuppressive-induced osteoporosis, osteoporosis due to renal failure, inflammatory osteoporosis, osteoporosis followed by Cushing's syndrome, rheumatoid osteoporosis), and
3) metastatic bone, hypercalcemia, Paget's disease, bone loss (e.g., alveolar bone loss, mandibular bone loss, childhood idiopathic bone loss), bone disease of osteonecrosis; and

are useful as agents for accelerating bone formation and/or curing after bone operations (for example, bone formation after fracture, bone formation after bone grafting, bone formation after operation for artificial joint, bone formation after spinal fusion, bone formation after other bone regeneration, etc.) and for alternative treatment for bone grafting.

WO00/03980 discloses that compound (I) is useful as an agent for binding to EP₄ receptor.

WO01/37877 discloses that an EP₄ receptor agonist of compound (I) is useful for treatment for bone diseases, in which, however, only a general description relating to local administration of the compound is given. Specifically, whether local administration of a sustained release preparation comprising EP₄ receptor agonist is useful for treatment for bone diseases has not been experimentally demonstrated.

JP-A-2001-181210 discloses that local administration of an EP₄ receptor-selective agonist is useful for treatment for bone diseases, which, however, is not experimentally demonstrated.

Until now, some applications of EP₄-agonistic compounds to therapeutic agents for diseases associated with decrease in bone mass have been found. However, EP₄ agonists which have been found have a prostanoic acid skeleton, and it is considered that, when they are used in systemic administration, for example, in oral administration or intravenous administration (intravenous rapid injection, intravenous constant infusion), then it may cause some side effects, for example, influences on circulatory systems such as blood pressure depression or hear rate increase, or diarrhea. Accordingly, these compounds have a serious problem in that their safe dose is limited.

In treatment for bone diseases, it takes a lot of time for bone formation, and local administration of therapeutic agents to patients must be repeated many times. However, it is not always satisfactory in view of the burden for patients. Accordingly, it is preferable that a sustained release preparation is administered to the region of bone diseases at the frequently as less as possible.

Based on the above, medicine which act on the local region of bones directly and continuously are desired.

### DISCLOSURE OF THE INVENTION

The present inventors have considered that, when EP₄ agonist is locally administered, a therapeutic agent with no side effect in systemic administration can be prepared. Also, they have considered that, when EP₄ agonist capable of being formulated into a sustained release preparation for local administration is found, a therapeutic agent which has no side effect in systemic administration and is useful even when administered not so much frequently can be prepared.

In order to solve the above problems, the present inventors have found that the objects can be accomplished by formulating compound (I) into a sustained release film preparation together with a biodegradable polymer, and thus the present invention has been completed.

A film preparation with a biodegradable polymer that contains compound (I) as the active ingredient thereof is a novel sustained release preparation that has heretofore been quite unknown in the art.

Specifically, the present invention relates to a sustained release film preparation and a therapeutic agent for diseases associated with decrease in bone mass that are mentioned below.
1. A sustained release film preparation for local administration to the region of diseases associated with decrease in bone mass, which comprises, as an active ingredient, (11α,13E,15α)-9-oxo-11,15-dihydroxy-16-(3-methoxymethylphenyl)-17,18,19,20-tetranor-5-thiaprost-13-enoic acid methyl ester having the following formula (I) or a non-toxic salt thereof: 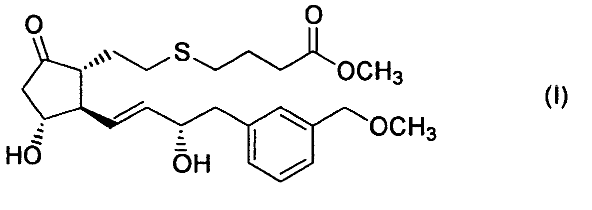
2. The sustained release film preparation according to the above 1, which has a film substrate comprising a biodegradable polymer.
3. The sustained release film preparation according to the above 2, wherein the biodegradable polymer is at least one polymer selected from the group consisting of a fatty acid ester polymer or copolymer, a polyacrylate, a polyhydroxybutyric acid, a polyalkylene oxalate, a polyorthoester, a polycarbonate and a polyamino acid.
4. The sustained release film preparation according to the above 3, wherein the fatty acid ester polymer or copolymer is a graft, block, alternating and/or random copolymer which comprises one or at least two of a polylactic acid, a polyglycolic acid, a polycitric acid, a polymalic acid, a poly-ε-caprolactone, a polydioxanone, a polyphosphagen, a poly-α-cyanoacrylate, a poly-β-hydroxybutyric acid, a polytrimethylene oxalate, a polyorthoester, a polyorthocarbonate, a polyethylene carbonate, a poly-γ-benzyl-L-glutamic acid, and a poly-L-alanine.
5. The sustained release film preparation according to the above 4, wherein the fatty acid ester polymer or copolymer is a polylactic acid, a polyglycolic acid or a lactic acid-glycolic acid copolymer.
6. The sustained release film preparation according to the above 5, wherein the polylactic acid or lactic acid-glycolic acid copolymer comprises lactic acid selected from L-lactic acid and poly-DL-lactic acid.
7. A therapeutic agent for local administration for diseases associated with decrease in bone mass, which comprises the sustained release film preparation according to any one of the above 1 to 6.
8. The therapeutic agent according to the above 7, wherein the disease associated with decrease in bone mass is primary osteoporosis (osteoporosis followed by aging, postmenopausal primary osteoporosis, osteoporosis followed by ovariectomy), secondary osteoporosis (glucocorticoid-induced osteoporosis, hyperthyroidism-induced osteoporosis, immobilization-induced osteoporosis, heparin-induced osteoporosis, immunosuppressive-induced osteoporosis, osteoporosis due to renal failure, inflammatory osteoporosis, osteoporosis followed by Cushing's syndrome, rheumatoid osteoporosis), metastatic bone, hypercalcemia, Paget's disease, bone loss (alveolar bone loss, mandibular bone loss, childhood idiopathic bone loss), bone disease of osteonecrosis, bone formation after operation for bone (bone formation after fracture, bone formation after bone grafting, bone formation after operation for artificial joint, bone formation after spinal fusion, bone formation after the other bone regeneration), or alternative treatment for bone grafting.

### BRIEF DESCRIPTION OF THE DRAWINGS

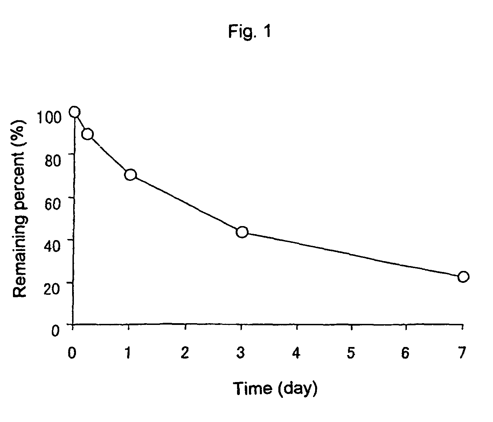

Fig. 1 is a graph showing a time-dependent remaining percent of compound (I) in a film preparation of the present invention.

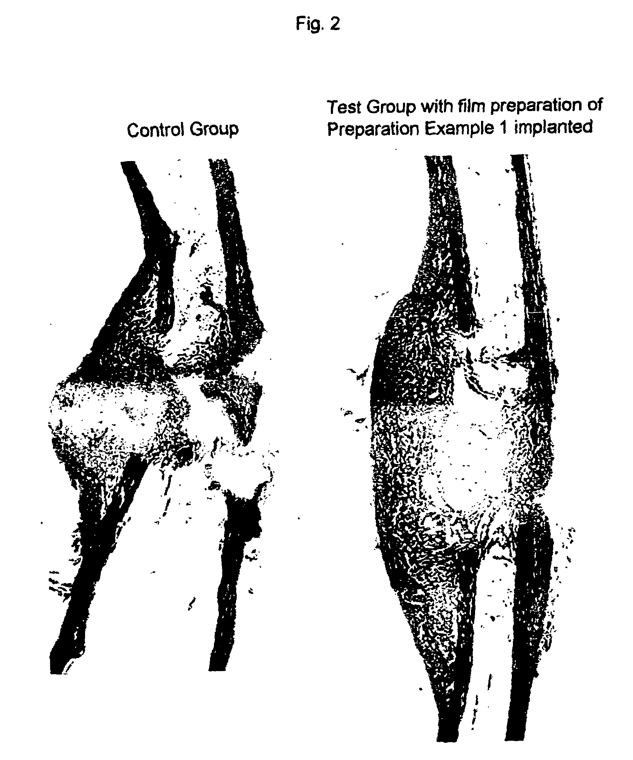

Fig. 2 is photographs substitutive for drawings, showing a tissue piece of an ulna-defective region of a rabbit of a control group after 3 weeks in evaluation for osteoanagenesis in the bone-defective region, and a tissue piece thereof of a test group after 3 weeks in which the preparation produced in Preparation Example 1 was implanted in the ulna-defective region of a rabbit.

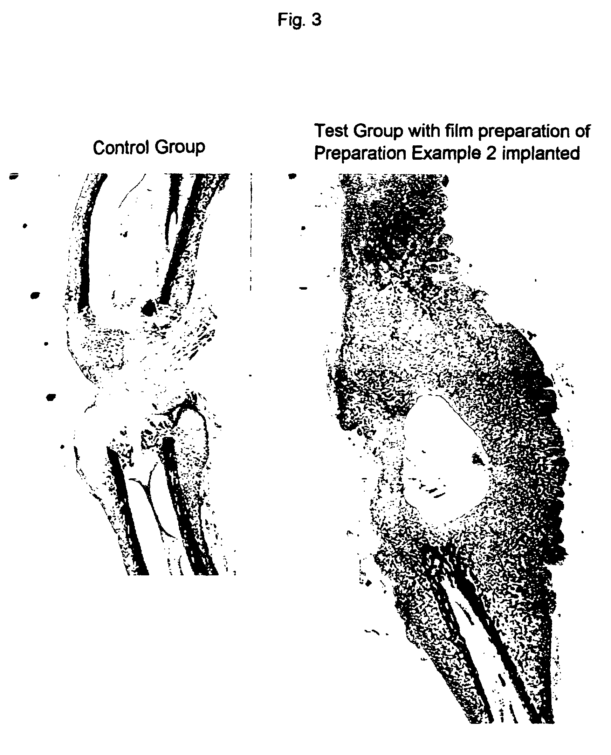

Fig. 3 is photographs substitutive for drawings, showing a tissue piece of an ulna-defective region of a rabbit of a control group after 3 weeks in evaluation for osteoanagenesis in the bone-defective region, and a tissue piece thereof of a test group after 3 weeks in which the preparation produced in Preparation Example 2 was implanted in the ulna-defective region of a rabbit.

### DETAILED DESCRIPTION OF THE INVENTION

The active ingredient of the sustained release film preparation of the present invention is (11 α,13E,15α)-9-oxo-11,15-dihydroxy-16-(3-methoxymethylphenyl)-17,18,19,20-tetranor 5-thiaprost-13-enoic acid methyl ester represented by formula (I) (compound (I)) or a non-toxic salt thereof.

The non-toxic salt includes solvates. The solvates are preferably non-toxic and soluble in water. Suitably, the solvates are those with water or an alcoholic solvent (e.g., ethanol).

The biodegradable polymer for the film substrate for the sustained release film preparation of the present invention includes fatty acid ester polymers or copolymers, polyacrylates, polyhydroxybutyric acids, polyalkylene oxalates, polyorthoesters, polycarbonates and polyamino acids. One or more of these can be used herein, singly or as combined. The fatty acid ester polymers or copolymers include polylactic acid, polyglycolic acid, polycitric acid, polymalic acid, poly-ε-caprolactone, polydioxanone, polyphosphagen and the like, and graft, block, alternating and random copolymers which contain two or more such components. One or more of these may be used herein, singly or as combined. In addition, they include poly-α-cyanoacrylates, poly-β-hydroxybutyric acids, polytrimethylene oxalates, polyorthoesters, polyorthocarbonates, polyethylene carbonates, poly-γ-benzyl-L-glutamic acids and poly-L-alanines. Copolymers of two or more of these components or those with the above-mentioned components, as well as one or more of these components as their mixtures may be used herein. Preferred are polylactic acid, polyglycolic acid and lactic acid-glycolic acid copolymer.

Lactic acid used in the polylactic acid and lactic acid-glycolic acid copolymer for use herein includes L-lactic acid and DL-lactic acid.

Preferably, the biodegradable polymer for use in the present invention has a mean molecular weight of about 2,000 to about 800,000, more preferably about 5,000 to about 200,000. For example, polylactic acid for use herein preferably has a weight-average molecular weight of about 5,000 to about 100,000, and more preferably about 6,000 to about 50,000. The polylactic acid may be produced according to a per-se known method.

In lactic acid-glycolic acid copolymer for use herein, the compositional ratio of lactic acid to glycolic acid may fall between about 100/0 and about 0/100 (w/w), but preferably between about 90/10 and about 30/70 (w/w) depending on the object of the copolymer. Preferably, the weight-average molecular weight of the lactic acid-glycolic acid copolymer is from about 5,000 to about 100,000, and more preferably from about 10,000 to about 80,000. The lactic acid-glycolic acid copolymer may be produced according to a per-se known method.

In the specification, the weight-average molecular weight of a polymer indicates the molecular weight thereof in terms of polystyrene, measured through gel permeation chromatography (GPC).

The amount of the above-mentioned biodegradable polymer for use herein may be varied in any desired manner in consideration of the intensity of the pharmacological activity of compound (I) and the release rate of compound (I) from the preparation, within a range within which the objective of drug administration can be attained.

For example, the amount of the biodegradable polymer may be in a ratio (by weight) of about 0.2 to about 10,000 times the pharmacological active substance (compound (I)), preferably in a ratio (by weight) of about 1 to about 1,000 times, and more preferably in a ratio (by weight) of about 1 to about 100 times.

The preparation method for the film preparation of the present invention is not specifically defined. For example, the film preparation may be prepared by dissolving the above-mentioned biodegradable polymer and compound (I) in an organic solvent and then forming it into a film through evaporation to dryness, air-drying or freeze-drying; or dissolving the biodegradable polymer in an organic solvent, separately dissolving compound (I) in water or a solvent not miscible with the organic solvent, then mixing the two through emulsification, and freeze-drying it into a film; or dissolving the biodegradable polymer and compound (I) in a suitable solvent, adding thereto a thickener (e.g., cellulose, polycarbonate), and gelling it into a film.

### INDUSTRIAL APPLICABILITY

### Application to Medicine :

Since compound (I) acts specifically and strongly on a PGE₂ receptor subtype EP₄, it is useful for treatment for primary osteoporosis (osteoporosis followed by aging, postmenopausal primary osteoporosis, osteoporosis followed by ovariectomy), secondary osteoporosis (glucocorticoid-induced osteoporosis, hyperthyroidism-induced osteoporosis, immobilization-induced osteoporosis, heparin-induced osteoporosis, immunosuppressive-induced osteoporosis, osteoporosis due to renal failure, inflammatory osteoporosis, osteoporosis followed by Cushing's syndrome, rheumatoid osteoporosis), metastatic bone, hypercalcemia, Paget's disease, bone loss (alveolar bone loss, mandibular bone loss, childhood idiopathic bone loss), bone disease of osteonecrosis, bone formation after operation for bone (bone formation after fracture, bone formation after bone grafting, bone formation after operation for artificial joint, bone formation after spinal fusion, bone formation after the other bone regeneration), or alternative treatment for bone grafting.

### Local Application:

The sustained release preparation of the present invention is used for direct, sustained release and local administration of compound (I) to affected sites. One embodiment of administration is an implant preparation.

The release period of compound (I) from the sustained release film preparation of the present invention may vary, depending on the type and the amount of the biodegradable polymer in the preparation. In general, however, the sustained release period of the preparation may be from 1 week to 3 months though depending on the objective thereof, and therefore, the preparation may be used for diseases associated with decrease in bone mass. Above all, the preparation of the invention is especially effective for cases that are desired to be avoided from frequent drug administration and are desired to undergo once drug administration for curing promotion continuously, for example, for cases of fracture whose affected parts are often fixed and covered with plaster.

The dose of the pharmaceutical ingredient from the sustained release film preparation of the invention may vary, depending on the drug-release duration of the preparation and on the animals for drug administration thereto, and an effective amount of compound (I) shall be administered from it. For example, when the preparation is used for fractured sites, then one dose thereof may be from about 0.001 mg to about 500 mg per adult (body weight 50 kg) in terms of the active ingredient of the preparation, but preferably from about 0.01 mg to about 50 mg per adult, and the preparation may be administered once for a week or three months.

### BEST MODE FOR CARRYING OUT THE INVENTION

The present invention is described below with reference to Preparation Examples, Preparation Test Example, and Example for osteoanagenesis evaluation, but the present invention should not be limited by following description.

### Preparation Example 1

A chloroform solution (1 ml) containing compound (I) (1 mg) was put into a chloroform solution (1 ml) containing 4.76% by weight of poly-L-lactic acid (weight-average molecular weight: 8,200 - hereinafter referred to as "PLLA") prepared by a known method, and mixed. The resulting mixture was poured and spread onto a glass dish having a diameter of 3 cm, and was allowed to stand horizontally for 3 or 4 days at room temperature to form a polylactic acid film preparation (0.1 mm thick).

### Preparation Example 2

A chloroform solution (1 ml) containing compound (I) (1 mg) was put into a chloroform solution (1 ml) containing 4.76% by weight of DL-lactic acid/glycolic acid copolymer (DL-lactic acid : glycolic acid =1 : 1 (mol%), weight-average molecular weight 53,114, produced by Purac - hereinafter referred to as "PDLGA"), and mixed. The resulting mixture was poured and spread onto a glass dish having a diameter of 3 cm, and was allowed to stand horizontally for 3 or 4 days at room temperature to form a polylactic acid-type film preparation (0.1 mm thick).

### Preparation Test Example 1

To the film prepared in Preparation Example 1 (about 5 mg), 1/15 M phosphate buffer (pH 6.8) containing 0.2% Tween-80 (1 ml) was added, followed incubation at 37°C. Its sample was centrifuged at the appropriate intervals to remove the supernatant from it, and the content of compound (I) in the resulting pellets was determined by high-performance liquid chromatography (HPLC). Assuming that the total, of compound (I) in the centrifugal supernatant and the pellets at the initial of the test was 100%, the remaining percent of compound (I) in the tested film was calculated.

Fig. 1 shows the time-dependent remaining percent of compound (I) in the film preparation tested according to the above method.

Fig. 1 confirms sustained release of compound (I) from the film preparation.

### Example for evaluation of osteoanagenesis of film preparations

Osteoanagenesis in bone-defective regions was evaluated according to the test method mentioned below.

### Preparation of bone-defective models:

Ulna-defective rabbit models were prepared. Each Japanese white rabbits having a body weight of 3 kg (bought from Shimizu Experimental Materials) was systemically anesthetized by administering Nembutal (3 mg/kg) thereto via their auditory vein. The arm of each rabbit was shaven and opened, and the muscle was removed from it to expose the ulna. The ulna was cut off along with the periosteum around it with scissors, and a 10 mm-gap bone loss was formed in each rabbit. The polylactic acid-type film preparation of Preparation Example 1 or 2 was implanted into the bone-defective region of each rabbit. The muscle was rearranged and the wounded skin was sutured. For control, other rabbits were operated in the same manner in which, however, the film preparation was not implanted. Three weeks after the implantation, the rabbits were sacrificed by administering excess Nembutal thereto via their auditory vein. The ulna was taken out of each rabbit and fixed with formalin. This was stained with hematoxylin-eosin, and the stained tissue was observed. For bone density determination, Dichroma Scan DC-600 (by Aloka) was used. According to double energy X-ray absorptiometry at two energy levels of 27 KeV and 53 KeV, the range (2 mm × 10 mm) of the regenerated bone of each rabbit was scanned with the device.

The test results with the above-mentioned models are shown in Table 1 and Figs. 2 and 3.

**Table 1**

| Preparation Example | Bone Density (mg/cm²) |
|---|---|
| Control | 181 (n = 4) |
| Preparation Example 2 | 327 (n = 2) |

From Table 1 and Figs. 2 and 3, it is clear that the compound (I)-containing film preparation implanted in the bone-defective region of the rabbit models induced bone formation in the region.

## Claims

1. A sustained release film preparation for local administration to the region of diseases associated with decrease in bone mass, which comprises, as an active ingredient, (11α,13E,15α)-9-oxo-11,15-dihydroxy-16-(3-methoxymethylphenyl)-17,18,19,20-tetranor-5-thiaprost-13-enoic acid methyl ester having the following formula (I) or a non-toxic salt thereof:

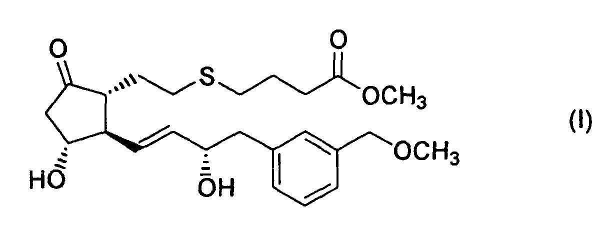

2. The sustained release film preparation according to claim 1, which has a film substrate comprising a biodegradable polymer.

3. The sustained release film preparation according to claim 2, wherein the biodegradable polymer is at least one polymer selected from the group consisting of a fatty acid ester polymer or copolymer, a polyacrylate, a polyhydroxybutyric acid, a polyalkylene oxalate, a polyorthoester, a polycarbonate and a polyamino acid.

4. The sustained release film preparation according to claim 3, wherein the fatty acid ester polymer or copolymer is a graft, block, alternating and/or random copolymer which comprises one or at least two of a polylactic acid, a polyglycolic acid, a polycitric acid, a polymalic acid, a poly-ε-caprolactone, a polydioxanone, a polyphosphagen, a poly-α-cyanoacrylate, a poly-β-hydroxybutyric acid, a polytrimethylene oxalate, a polyorthoester, a polyorthocarbonate, a polyethylene carbonate, a poly-γ-benzyl-L-glutamic acid, and a poly-L-alanine.

5. The sustained release film preparation according to claim 4, wherein the fatty acid ester polymer or copolymer is a polylactic acid, a polyglycolic acid or a lactic acid-glycolic acid copolymer.

6. The sustained release film preparation according to claim 5, wherein the polylactic acid or lactic acid-glycolic acid copolymer comprises lactic acid selected from L-lactic acid and poly-DL-lactic acid.

7. A therapeutic agent for local administration for diseases associated with decrease in bone mass, which comprises the sustained release film preparation according to any one of claims 1 to 6.

8. The therapeutic agent according to claim 7, wherein the disease associated with decrease in bone mass is primary osteoporosis, secondary osteoporosis, metastatic bone, hypercalcemia, Paget's disease, bone loss, bone disease of osteonecrosis, bone formation after operation for bone, or alternative treatment for bone grafting.
